# EUROPEAN PATENT APPLICATION

(11) **EP 1 347 041 A1**
(43) Date of publication of application: **24.09.2003**
(21) Application number: 02090118.7
(22) Date of filing: 21.03.2002
(51) Int. Cl.: C12N 5/06

(54) **Mammalian permanent growth factor-independent cell line and method of its production**

(71) Applicant: Universitätsklinikum Benjamin Franklin der Freien Universität Berlin, 12200 Berlin (DE)
(72) Inventor: Notter, Michael Dr., 12205 Berlin (DE)
(74) Representative: Ziebig, Marlene, Dr. Dipl.-Chem.

(57) **Abstract**

The invention relates to a mammalian permanent growth factor-independent cell line constitutively overexpressing the tyrosine kinase receptor *c-kit* and/or proteins derived thereof, the use of the cell line and a method of producing the cell line.

## Description

The present invention relates to a mammalian permanent growth factor-independent cell line constitutively overexpressing the tyrosine kinase receptor *c-kit* and/or proteins derived thereof, the use of the cell line and a method of producing the cell line.

Despite advantages of high-dose chemotherapy and stem cell transplantation, cases of acute myeloid leukemia (AML) with complex cytogenetic abnormalities including monosomy 7 continue to have poor therapeutic outcomes. Relapse rates following allogeneic bone marrow transplantation exceed 70 % illustrating the need for new treatment modalities in this patient subgroup.

The tyrosine kinase *c-kit*, which is the receptor for stem cell factor (SCF), is expressed in 70-80 % of AML patients. In the majority of these cases, the role of *c-kit* in transformation and tumor progression is unknown, although activating mutations have been described occasionally (Kitayama *et al.,* Blood, 85: 790-798, 1995). Nevertheless, the bulk of evidence argues against *c-kit* being an independent prognostic factor in AML-patients. In particular, it remains unclear, if *c-kit* positively reflects part of a normal stem cell differentiation program, or if it essentially contributes to transformation or progression in that disease. Investigating mechanisms behind states of dormancy of the host immune system due to tolerance, T cell anergy or poor immunogenicity may prove instrumental to develop new immunotherapeutic strategies. However, the *c-kit* receptor expressed on AML blasts generally appears to initiate a signaling cascade when dimerized by its ligand SCF (Broudy *et al*., Blood, 80:60-67, 1992).

It is an object of the present invention to establish a cell line suitable for being used for screening of drugs for influencing or interrupting the SCF-related signaling cascade associated in transformation or progression in AML. It is another object of the invention to provide a method for producing this cell line .

This and other objects of the present invention are accomplished by a mammalian permanent growth factor independent cell line constitutively overexpressing the surface marker tyrosine kinase receptor (*c-kit*) and/or proteins derived of *c-kit*. According to the invention proteins derived of *c-kit* are proteins with 80% homology to *c-kit*, preferably with 90% homology to *c-kit*. Overexpression herein is defined as *c-kit* expression that ranges in the third decade of the mean fluorescence intensity scale, as determined by flow cytometry using antibodies directly conjugated to phycoetythrin. Overexpression of c-kit is uncommon in AML (Cole et *al.,* Leukemia, 10: 288-296, 1996).

According to a preferred embodiment of the invention at least 80 %, particularly at least 85 %, preferably more than 90 % of the cells of the cell line have the ability to express *c-kit*.

The cell line of the present invention is preferably derived from a patient with myeloid leukemia, more preferably from a patient with acute myeloid leukemia (AML). In particular, the cell line may be of the subtype M5a according to the French American British classification, and is preferably characterized by the complex karyotype monosomy 7, t(2;11) (q31:p13), t(10;12)(q24;q24). The region 7(q22q35) is supposed to encode tumor suppressing genes (Liang *et al.,* Proc. Natl. Acad. Sci. USA, 95: 3781-3785, 1998).

The cell line according to the present invention is further preferentially characterized by poor immunogenicity. In particular, the present cell line is not able to stimulate T cell proliferation despite the presence of costimulatory cytokines.

According to another aspect of the invention, the cell line is actively immunosuppressive. In particular, the cell line has the ability of suppressing T cell proliferation, when the cell line is used as a third party stimulator.

The cell line is further preferred to have the ability of differentiating into dendritic cells (DCs) in the presence of cytokines, wherein the cytokines may be recombinant cytokines and/or cytokines produced by stimulated T-cells. Particularly, DCs derived from the cell line according to the present invention are not able to stimulate T cell proliferation.

According to another aspect of the invention, the cell line is the cell line deposited at the Deutsche Sammlung von Mikroorganismen und Zellkulturen (DSMZ) in Braunschweig under deposit No. ACC2534.

The invention further relates to proteins derived from the cell line according to the present invention, these proteins preferably include the tyrosine kinase receptor *c-kit* and/or proteins derived thereof.

Due to its ability of oxerexpressing *c-kit*, the cell line according to the present invention can advantageously be used for producing and purifying this surface receptor protein and secondary proteins attached to it under circumstances of malignant transformation. It can also be used as a tool in drug development in the setting of screening novel compounds targeting the *c-kit* signaling pathway in various malignancies.

A further extraordinary valuable aspect of the invention concerns the use of the cell line for establishing tumors in mammalians, especially in mice, and for screening agents acting with tumor cells *in vivo* and/or *in vitro.*

The invention further relates to a method of producing the cell line. The method comprises obtaining a whole blood sample of a mammalian individual, preferably suffering of AML; separating peripheral blood mononuclear cells (PBMNC) from the whole blood sample; inoculation of the PBMNC in an enriched culture medium and continuously culturing the cells for a first period of time; replacing the enriched growth medium by a standard culture medium and continuously culturing the cells for a second period of time. This procedure was shown to result in a cell line having the above indicated characteristics.

Further preferred embodiments of the invention are given by the dependent claims.

The invention is further explained in more detail by examples referring to the Figures.

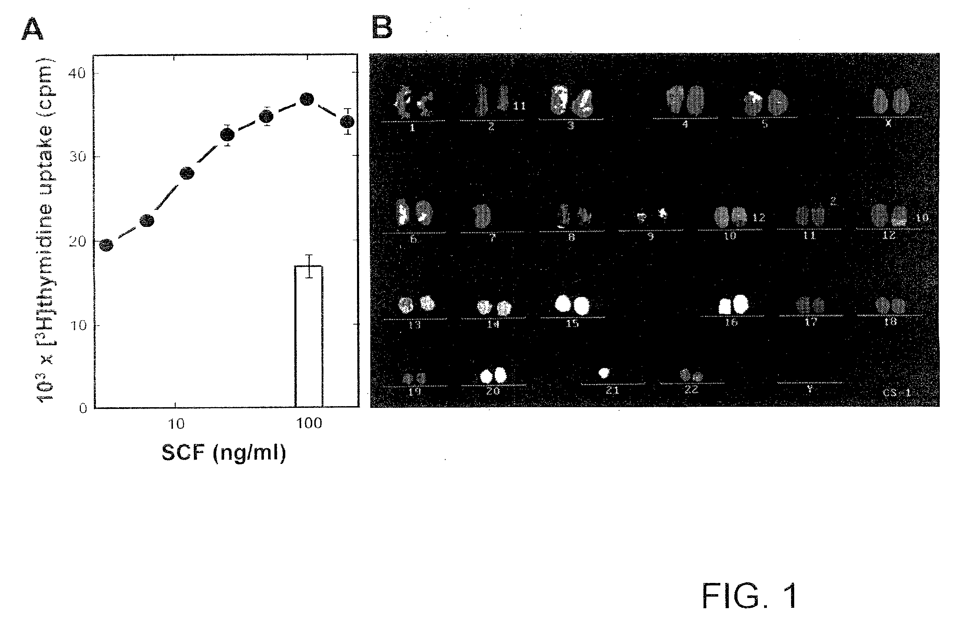

- Figure 1: *Growth characteristics and karyotype of CS-1 cells.* (A) CS-1 cells were incubated without additional cytokines (white bar) or with recombinant SCF, respectively (black circles). During the last 4 of 96 hours microcultures received [6-³H]thymidine. Each point represents the mean value and the standard derivation (SD) of six microcultures. (B) CS-1 cells at day 1,235 were subjected to 24-color fluorescence-*in situ*-hybridisation.

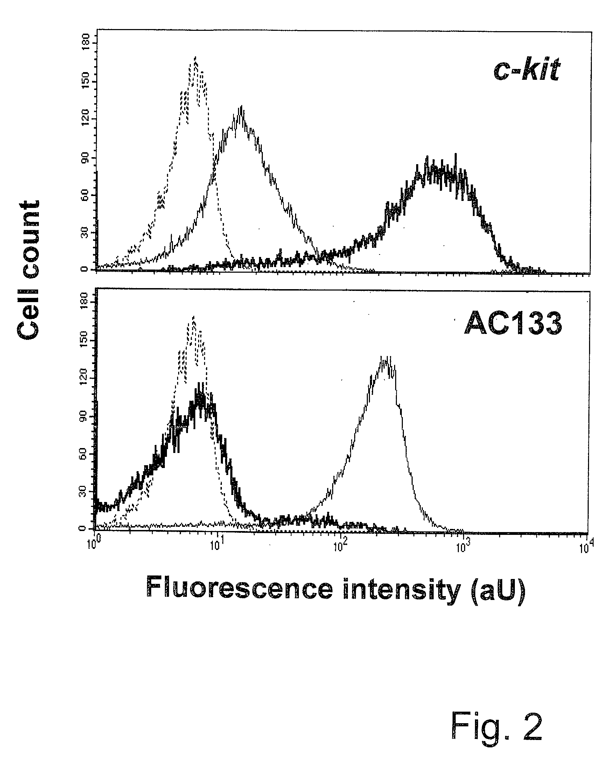

- Figure 2: *Comparison of c-kit and AC133 expression by normal CD34*^{*+*} *cells and CS-1*. Non-malignant CD34⁺ progenitor cells (light line) and CS-1 cells (bold line), respectively, were incubated with directly conjugated mAb specific for *c-kit* or AC133. Irrelevant murine IgG1 served as negative control (dotted line). Binding to viable cells was detected by flow cytometry.

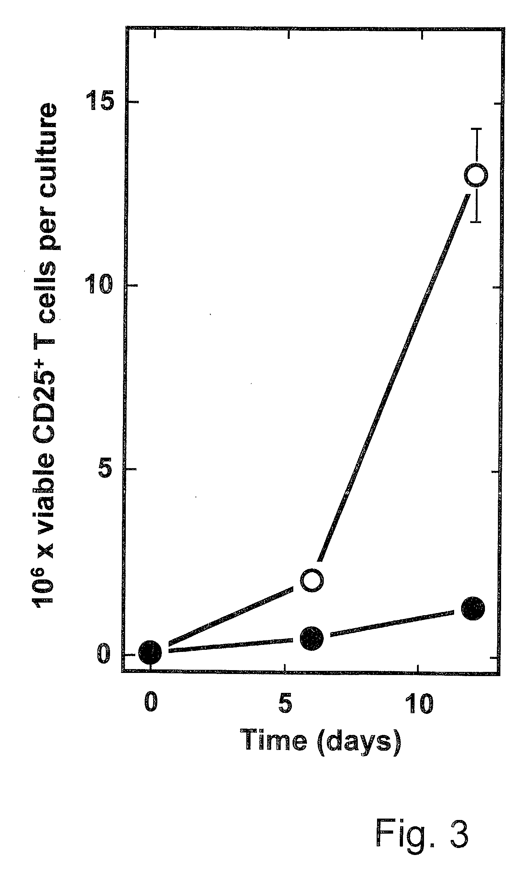

- Figure 3: *Activation of allogeneic T cells by CS-1 cells.* Resting HLA non-matched responder T cells were incubated with Mitomycin C-inactivated CS-1 cells (black circles) or EBV-B cells (white circles), respectively, in the presence of soluble IL-2. Cultures were restimulated weekly. Viable CD25⁺ T cell counts were determined by flow cytometry. Each point represents the mean value and the SD of six microcultures.

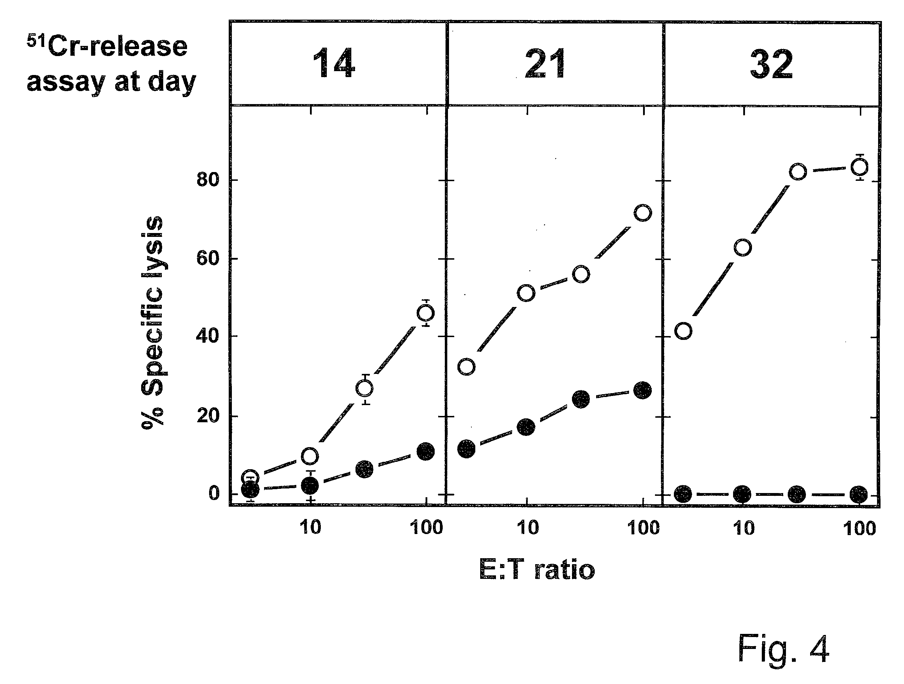

- Figure 4: *Induction of allogeneic cytotoxic T cells by CS-1 cells.* Allogeneic, HLA non-matched responder T cells were stimulated as described in Figure 3. Stimulator-specific cell-mediated cytotoxicity was detected in a ⁵¹Cr-release assay with the appropriate target CS-1 cells (black circles) and EBV-B cells (white circles) at different E:T ratios. No CS-1 activated T cells were available at day 32.

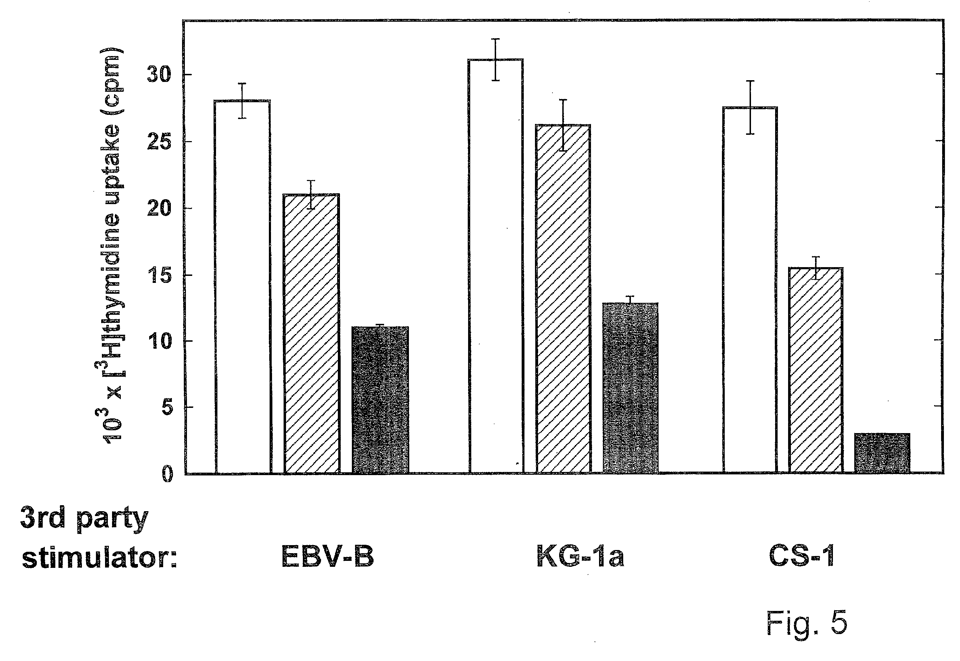

- Figure 5: *Immunosuppression by CS-1*. Allogeneic resting T cells and Mitomycin C-inactivated normal EBV-B cells were incubated with various numbers of inactivated EBV-B, KG-1a and CS-1 cells as third party cells, respectively. Microcultures received [6-³H]thymidine during the last 18 hours. The ratio of third party to EBV-B cells was 0.4:1 (white bars), 1.3:1 (hatched bars) or 4:1 (black bars). Each bar represents the mean value and the SD of [6-³H]thymidine uptake of three independent microcultures.

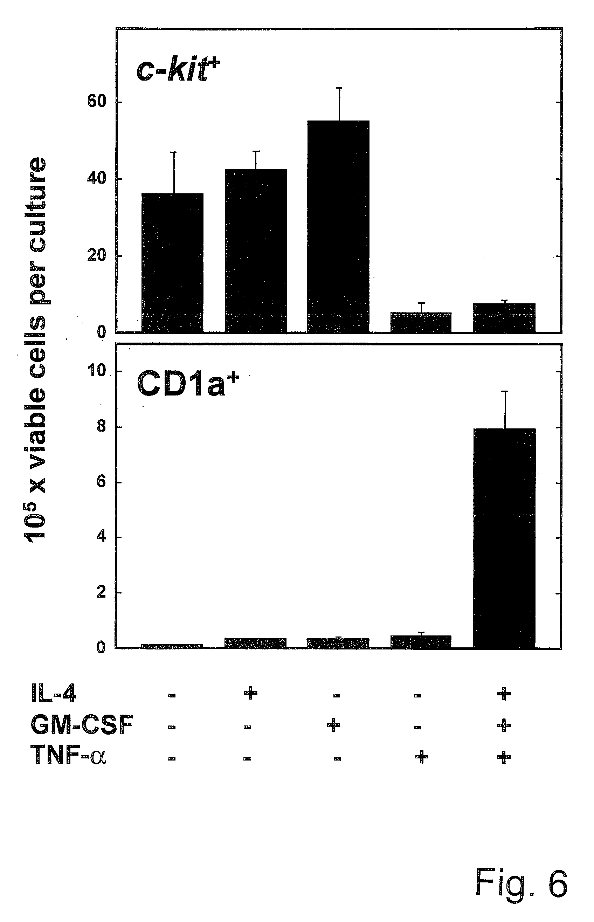

- Figure 6: *Cytokine-induced DC differentiation of CS-1*. CS-1 cells from continuous *in* vitro-culture were incubated with 10 ng/ml IL-4, 100 ng/ml GM-CSF and 10 ng/ml TNF-α, alone or in a cocktail. After 10 days the total amount of viable cells expressing the progenitor marker *c-kit* and the DC marker CD1a, respectively, was determined by flow cytometry. Each bar represents the mean value and the SD of six microcultures.

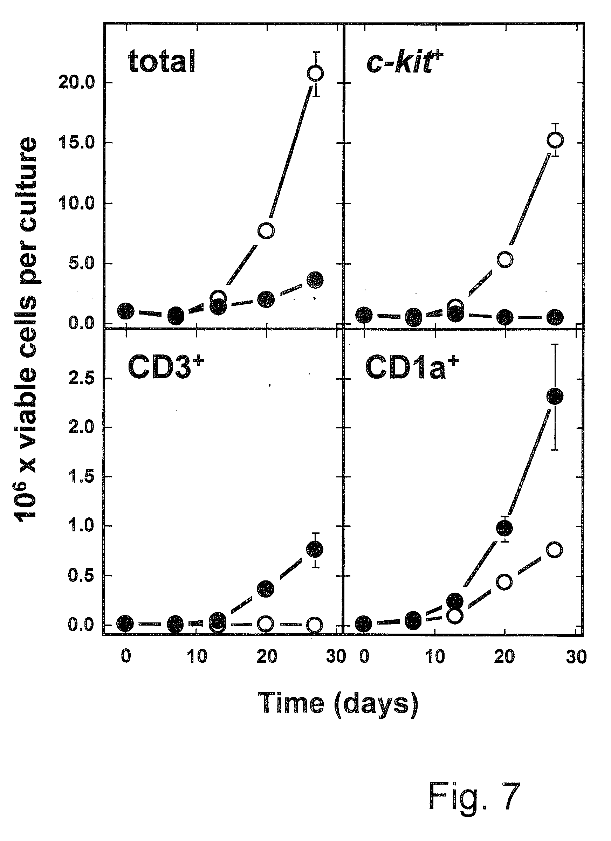

- Figure 7: *DC differentiation of CS-1 during autologous T cell activation.* PBMNC obtained at the time of relapse were cultured with 100 ng/ml murine IgG2a (white circles) or with 100 ng/ml anti CD3 mAb (clone OKT3) and 50 U/ml IL-2 (black circles). The total amount of viable cells and the number of cells expressing *c-kit*, the T cell marker CD3 and the DC marker CD1a, respectively, were determined by flow cytometry. Each point represents the mean value and the SD of six microcultures.

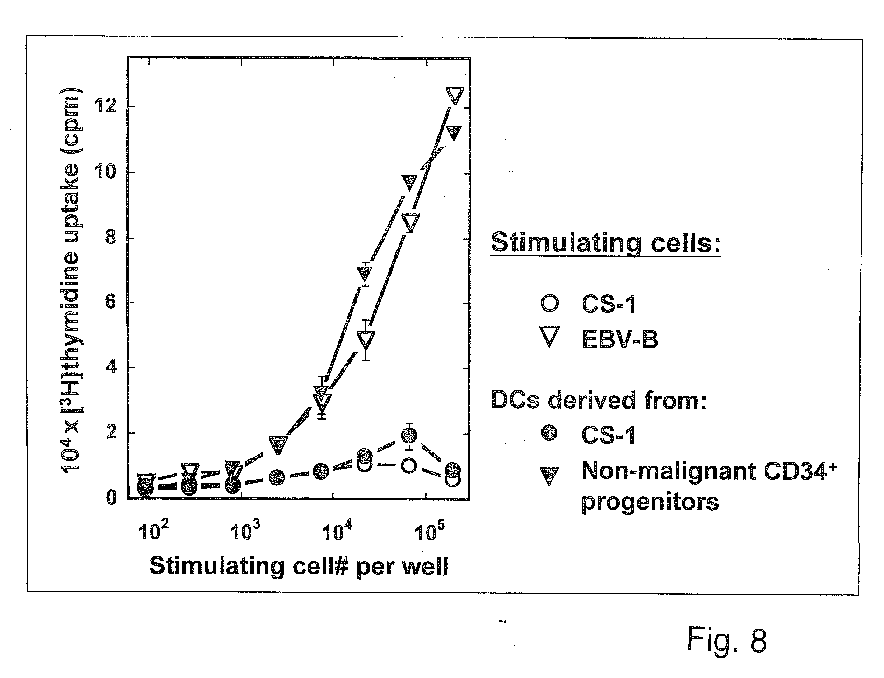

- Figure 8: *Failure of allogeneic T cell activation by CS-1-derived DCs.* HLA non-matched allogeneic T cells were incubated for 96 hours with increasing stimulating cell numbers and with [6-³H]thymidine during the last 4 hours. Each bar represents the mean value and the SD of three microcultures.

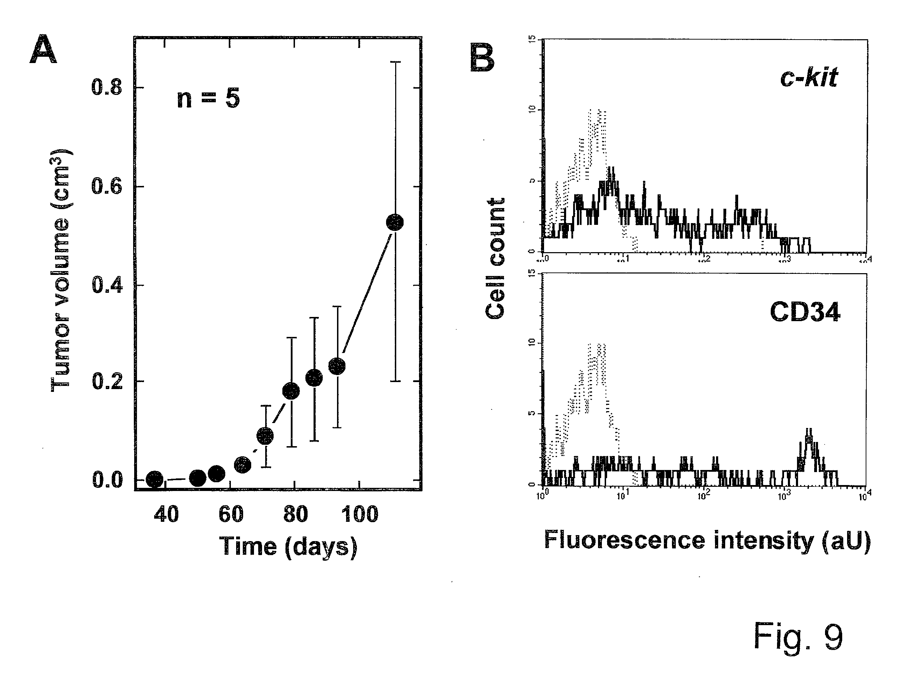

- Figure 9: *CS-1 tumor growth in NOD*/*SCID mice.* (A) Freshly thawed *in* vivo-passaged tumor tissue was transplanted *s.c.* into the lateral abdomen of NOD/SCID mice. Tumor growth was monitored over time. Each point represents the mean value and the SD of tumor size in five mice. (B) Solid *s.c.*-tumors excised from NOD/SCID mice inoculated with first passage CS-1 cells four month before. (C) 2x10⁴ cells from suspended solid *s.c.*-tumor tissue in second passage were incubated with mAb specific for human *c-kit* and CD34, respectively (bold lines). Binding of the directly conjugated mAbs was detected by flow cytometry. Irrelevant murine IgG1 served as negative control (dotted lines).

Before explaining the preferred embodiments of the present invention by example, some general materials and methods used herein are described.

*Media and cell lines.* Standard culture medium consisting of RPMI1640 with 10 % fetal bovine serum (FBS, both from Life Technologies; Gaithersburg, MD, USA), 1 mM sodium pyruvate and 2 mM L-glutamine (both from Biochrom, Berlin, Germany) was used to maintain the CS-1 cell line, the KG-1a cell line and normal Epstein Barr virus-transformed (EBV)-B cells. CS-1 cells were initially cultured in an enriched culture medium containing 20 % FBS, 2 mM L-asparagine and 2 mM L-arginine (both from Life Technologies) and subsequently shifted to the standard culture medium after 75 days of continuous *in* vitro-culture. Cell density varying between 2x10⁵ and 1x10⁶ per ml was adjusted to allow optimal cell viability. Cells were cultured in a humidified atmosphere at 37 °C and 5.5 % CO₂. Bisbenzimide staining for mycoplasma contamination (Biochrom) was performed every 2-4 weeks. Positive cells were discarded. Cells were routinely checked by flow cytometry to monitor potential phenotypic variation over time.

*Patient characteristics.* Heparinzed whole blood samples were obtained from a patient with AML of the subtype M5a according to the French American British classification after a relapse following stem cell transplantation of an unrelated donor. Normal CD34⁺ progenitor cells were prepared according to the Miltenyi (Sunnyvale, CA, USA) standard protocal for positive magnetic cell separation of CD34⁺ cells from peripharal blood of a non-hematopoietic cancer patient after mobilization by chemotherapy and granylocyte macrophage-colony stimulating factor (GM-CSF) treatment. Normal allogeneic T cells were prepared from heparinized whole blood samples of healthy donors.

*Flow cytometry.* 2x10⁵ cells were washed once with 0.5 % bovine serum albumine (DADE Behring, Marburg, Germany) in phosphate buffered NaCl solution consisting of 0.21 g/l monobasic potassium phosphate, 9 g/l sodium chloride and 0.726 g/l dibasic sodium phosphate heptahydrate, (pH 7.4; Biochrom). Non-specific antibody binding was blocked by heat inactivated rabbit serum (Sigma, St. Louis, MS). If not noted otherwise, all monoclonal antibodies (mAbs) used were directly conjugated with phycoerythrin or fluoresceinisothiocyanate. The following mAbs specific for human antigens were used: CD1a/clone BL6, CD4/clone 13B8.2, CD8/clone B9.11, CD13/clone SJ1D1, CD14/clone RMO52, CD15/clone 80H5, CD19/clone J4.119, CD25/clone 33B3.1, CD33/clone D3HL60-251, CD38/clone T16, CD54/clone 84H10, CD56/clone N901, CD58/clone AICD58, CD65/clone 88H7, CD80/clone MAB104, CD90/clone F15.42, c-kit(CD117)/clone 95C3, CD135/clone SF1.340, HLA-class I/clone B8.12.2 (all from Immunotech, Marseille, France), CD3/clone SK7, CD7/clone Leu7, CD34/clone 8G12, CD86/clone IT2.2, HLA-DR/clone L243 (all from Becton Dickinson, San Jose, CA, USA), FcγRI(CD64)/clone 32.2 (Medarex, Annandale, NJ, USA), and AC133/clone AC133-1 (Miltenyi). Irrelevant mAbs of the appropriate isotypes (all from Immunotech) served as negative controls. Viability testing of the cells was done using propidium iodine (ICN, Aurora, OH, USA) staining. For the evaluation with three color-flow cytometry the FACScan™ device and the CellQuest™ software (both from Becton Dickinson) were used.

*Mixed-lymphocyte-tumor-culture (MLTC).* Allogeneic healthy T cell donors were selected after HLA-typing to ensure disparity in at least two loci. PBMNC isolated from heparinized whole blood samples from these donors by density gradient centrifugation as described previously were subjected to nylon-wool filtration. Macrophages were depleted according to the Miltenyi standard protocol for negative magnetic cell separation using the FcγRI specific mAb. As checked by flow cytometry, the percentage of CD3⁺ T cells was more than 92 % with a CD8⁺/CD4⁺ ratio of 1:3.5. Macrophages (CD64⁺) and B-lymphocytes (CD19⁺) were below 1 %.

### 1. Isolation of primary leukemic cells and establishment of the CS-1 cell line.

Heparinized whole blood samples were obtained from a patient with acute myleoid leukemia (AML) of the subtype M5a according to the French American British classification after relapse following stem cell transplantation of an unrelated donor. The peripheral blood mononuclear cells (PBMNC) fraction from the heparinized whole blood was obtained by Hypague-Ficoll™ (Amersham Pharmacia, Little Chalfont, UK) density cell centrifugation (ρ=1.078 g/ml). Contaminating erythrocytes were lysed using a red cell lysis buffer consisting of 155 mM ammonium chloride, 10 mM potassium bicarbonate, and 1 mM EDTA (all from Merck, Darmstadt, Germany). Cell preparations contained more than 95 % of blast cells as assessed by flow cytometry using the CD34, *c-kit* and AC-133 specific monoclonal antibodies (mAbs). For long-term storage, blast cells preparations were frozen in multiple aliquots of 10⁷ cells per 2 ml FBS with 20 % dimethylsulfoxide (Serva, Heidelberg, Germany) over liquid nitrogen.

Freshly isolated leukemia cells derived from the relapsed AML-M5a patient or long-term storage preparations were inoculated at a density of 1x10⁶ cells per ml of the enriched culture medium. The enriched medium containing 20 % fetal bovine serum (FBS), 2 mM L-asparagine and 2 mM L-arginine was totally exchanged twice a week. Beginning at day 75 of continuous *in* vitro-culture, the enriched medium was replaced by standard culture medium containing 10 % FBS. After 300 days, inoculum cell density was lowered to 2-5x10⁵ cells per ml. This manipulation resulted in a permanent AML cell line subsequently referred to as CS-1.

### 2. Karyotype and growth characteristics of the CS-1 cell line

The CS-1 cells have a generation time of 57.6 ± 10.8 hours, which did not change over time. Under standard culture conditions, CS-1 cells in suspension have nearly uniform spherical blast-like morphology of single cells that occasionally form aggregates up to five cells. Until now cells were propagated for more than 1,300 days without any exogenous cytokines.

To investigate the influence of stem cell factor (SCF) on CS-1 a proliferation assay was applied. For this purpose, 5x10³ CS-1 cells were incubated for 96 hours with 200 µl standard culture medium (see above) in round bottom 96-well tissue culture plated (NUNC, Roskilde, Denmark). Starting from 200 ng/ml, soluble recombinant stem cell factor (SCF; Genzyme, Cambridge, MA, USA) was titrated to the microcultures. During the last 4 hours microcultures received 0.5 µCi [6-³H]thymidine (Amersham Pharmacia) per well. Cells were harvested to a glass fiber membrane and incorporated [6-³H]thymidine was detected by liquid scintillation counting (LKB Wallac, Turku, Finland). As seen in Figure 1A, addition of soluble SCF, caused dose-dependent growth stimulation. Saturating SCF concentrations (> 100 ng/ml) resulted in a 2.2-fold higher plateau of [6-³H]thymidine incorporation compared to medium control.

Morphological characteristics of the cell line were not affected by adding the soluble growth factor.

The karyotypes of the CS-1 cell line as well as of primary leukemic cells was determined from metaphase preparations by spectral karyotyping using 24 chromosome-specific painting probes in one fluorescence-in situ-hybdridisation experiment as described by Macville et *al.* (Histochem. Cell. Biol., 108: 299-305, 1997) overlayed by G-banding results. As shown in Figure 1B, the karyotypes of primary leukemic and of the CS-1 cell line were found to be identical. Besides the monosomy 7, the CS-1 karyotype includes the translocations t(2;11)(q31;p13) and t(10;12)(q24;q24). No detectable marker chromosomes were identified.

### 3. Immunophenotype of CS-1

CS-1 cells were maintained for 1,325 days in continuous *in* vitro-culture in standard culture medium. Expression of surface markers was confirmed by flow cytometry using directly conjugated mAb as explained above.

CS-1 cells show an aberrant phenotype (Table 1). Apart from the myeloid surface markers CD13 and CD33, the lymphatic antigens CD4 and CD7 are coexpressed by about 50 % of the CS-1 cells. The progenitor-associated antigens CD34, HLA-DR and the SCF-receptor *c-kit* are expressed in an asynchronous fashion. CS-1 cells showed a marked overexpression of the tyrosine kinase receptor *c-kit*, which is the receptor for the stem cell factor (SCF).

**Table 1:**

| Immunophenotype of CS-1. | | |
|---|---|---|
| Section | Surface marker | Positive cells [%] |
| Lymphoid | CD4 | 48.8 |
| | CD7 | 54.4 |
| Myeloid | CD13 | 100.0 |
| | CD14 | 1.4 |
| | CD15 | 5.6 |
| | CD33 | 99.8 |
| | CD65 | 36.7 |
| Progenitors | CD34 | 99.5 |
| | CD38 | 5.6 |
| | CD90 (Thy-1) | 9.1 |
| | CD117 (*c-kit*) | 99.9 |
| | AC133 | 12.4 |
| | CD135 (Flt3/flk2) | 4.2 |
| | HLA-DR | 97.4 |

To follow the development of *c-kit* expression during the course of initial *in* vitro-culture of isolated primary leukemic cells, 2x10⁵ cells from different points of continuous *in vitro*-culture were incubated with the *c-kit* specific mAb. Homogeneity of the cell population was confirmed by counterstaining with CD33 and CD34 specific mAb, respectively. Antibody binding was detected by flow cytometry as described above. CD33 and CD34 was expressed by > 98 % of all cells with MFI values remaining constant over time. Results of this test are summarized Table 2. Initially, about half of the AML blasts expressed *c-kit* and the mean fluorescence intensity (MFI) value was in the second decade. During two years of continuous *in vitro*-culture, a doubling in the percentage of *c-kit* expressing cells accompanied a continuous increase of the MFI value by one log, which did not change further. These data clearly indicate the success of the special culture conditions applied herein with respect to the expression rate of *c-kit* in AML blasts resulting in the new cell line CS-1 highly overexpressing the SCF-receptor protein.

**Table 2:**

| *c-kit* expression during continuous culture of CS-1. | | |
|---|---|---|
| Time [days] | *c-kit*^{*+*} cells [%] | MFI [aU] |
| 0 | 55.7 | 48.6 |
| 20 | 65.5 | 70.6 |
| 745 | 75.6 | 85.5 |
| 909 | 82.8 | 254.1 |
| 1,066 | 93.2 | 399.6 |
| 1,256 | 92.9 | 328.3 |
| 1,482 | 95.4 | 305.0 |

The expression profile of freshly isolated non-malignant CD34⁺ progenitor cells was compared to CS-1 after more than 1,000 days of culture. Normal CD34⁺ progenitor cells were prepared according to the Miltenyi (Sunnyvale, CA) standard protocol for positive magnetic cell separation of CD34⁺ cells from peripheral blood of a non-hematopoietic cancer patient after mobilization by chemotherapy and granulocyte macrophage-colony stimulating factor (GM-CSF). CD34⁺ progenitor cells and CS-1 cells were incubated with directly conjugated mAb specific for *c-kit* or AC133, respectively. Irrelevant murine IgGl served as negative control. Binding to viable cells was detected by flow cytometry as described above. Although *c-kit* was expressed by 61.1 % of the normal progenitor cells compared to 98.9 % of the CS-1 cells, the MFI value of the latter was increased by one log (MFI=22.5 arbitrary units (a.U.) versus 399.6 a.U.) , Figure 2. These data confirm the above observation that not only the percentage of *c-kit*^{*+*} cells is increased in CS-1 but also the density of this receptor on the cell surface. On the other hand, while 96.7 % of the normal CD34⁺ progenitor cells expressed AC-133, this early progenitor antigen was expressed by only 10.5 % of the CS-1 cells.

### 4. Immunogenicity of CS-1

To investigate the immunogenicity of CS-1 cells, allogeneic mixed-lymphocyte-tumor-cultures (MLTCs) were set up using allogeneic resting responder T cells and soluble IL-2. MLTCs were prepared as described above.

10⁶ resting responder T cells per well were incubated with 5x10⁵ stimulator cells and with 25 U/ml soluble interleukin-2 (IL-2; Eurocetus, Frankfurt, Germany) in a 24-well tissue culture plate (NUNC). CS-1 and EBV-B cells, respectively, that were inactivated for 30 minutes with 100 µg/ml Mitomycin C (Medac, Hamburg, Germany) served as stimulator cells. Every 7 days T cell proliferation and activation was evaluated by flow cytometry using the CD3, CD4, CD8, CD25 and CD56 specific mAbs, respectively. 2x10⁵ responder T cells per well were restimulated with 5x10⁵ of the appropriate stimulator cells and 25 U/ml soluble IL-2.

In addition, a *cell-mediated cytotoxicity assay* was performed. Starting at day 14, cytotoxic effector cells generated by the MLTC in 24-well tissue culture plates as described above were directly used in a ⁵¹chromium(Cr)-release assay performed at weekly intervals. 10⁶ target CS-1 and EBV-B cells, respectively, in 100 µl FBS were labeled for 2 hours at 37 °C and 5.5 % CO₂ with 100 µCi sodium [⁵¹Cr]chromate (Amersham Pharmacia). Cells were washed once with standard culture medium followed by a second incubation in 5 ml of the same medium for 30 minutes. Cells were again washed twice with medium. 5x10³ target cells were added to the effector cells and incubated for 4 hours at 37 °C and 5.5 % CO₂. Supernatants were harvested using a Skatron system (Skatron, Lier, Norway) and counted in a gamma counter (LKB Wallac). The percentage of specific ⁵¹Cr-release was calculated using the formula: percentage of specific release = (experimental release - spontaneous release) x 100/(maximum release after the addition of 100 µl 1 % TritonX-100 (Sigma Chemical, St. Louis, MO, USA) - spontaneous release). The spontaneous ⁵¹Cr-release as measured in the supernatant of the target cells cultured in medium alone had to be less than 10 % for results to be included into final analysis.

As shown in Figure 3, stimulation with EBV-B cells efficiently activated T cells as reflected by a significant expansion of CD25⁺ T cells on day 6 and 12. In contrast, CS-1 cells did not induce CD25⁺ T cell expansion. By weekly restimulation, T cell cultures could be maintained beyond 32 days when EBV-B cells were present, but only until day 21 using CS-1 stimulators. At an effector : target (E:T) ratio of 30:1 in ⁵¹Cr-release assays, maximum specific lysis of stimulator cells EBV-B-stimulated T cells was 82.4 ± 0.3 % on day 32 compared to 24.2 ± 0.7 % by CS-1-stimulated cultures on day 21, Figure 4. Additionally, CS-1-activated allogeneic T cells could not be cloned. These findings indicate that CS-1 cells have strongly reduced immunogenicity, although retaining in part sensitivity for T cell-mediated cytotoxicity.

### 5. Expression of costimulatory molecules

To address potential causes of the reduced immunogenicity of CS-1 cells, expression of costimulatory molecules was examined by flow cytometry (Table 3). For flow cytometry, 2x10⁵ cells were incubated with the appropriate mAb. Homogeneity of CS-1 and EBV-B cell population was confirmed by counterstaining with CD34 (CS-1: 98.5 %) and CD19 (EBV-B: 99.6 %), respectively.

Compared to normal EBV-B cells, the percentage of CD58⁺, CD80⁺ and CD86⁺ CS-1 cells was substantially lower, namely 64.1, 91.9 and 85.0 %, respectively. Although CD54, HLA-DR and HLA-class I was expressed on the majority of CS-1 cells, antigen density as indicated by the MFI value is reduced by 17.8-, 2.3- and 4.8-fold.

**Table 3:**

| Expression of T cell-costimulatory molecules by CS-1 in comparison to normal EBV-B-cells. | | | | |
|---|---|---|---|---|
| | Positive cells [%] | | MFI [aU] | |
| Antigen | CS-1 | EBV-B | CS-1 | EBV-B |
| CD54 (ICAM-1) | 87.8 | 99.7 | 57.9 | 1,029.0 |
| CD58 (LFA 3) | 35.6 | 99.7 | 18.0 | 108.6 |
| CD80 (B7/BB1) | 8.0 | 99.9 | 7.3 | 152.8 |
| CD86 (B70/B-2) | 14.8 | 99.8 | 11.0 | 386.8 |
| HLA-DR | 97.3 | 99.9 | 666.2 | 1,519.8 |
| HLA-class I | 98.8 | 99.8 | 317.6 | 1,542.0 |

### 6. Immunosuppression by CS-1

Immunosuppressive properties of CS-1 were tested by setting up allogeneic MLTCs (preparation described above) using EBV-B cells as stimulators and with CS-1, KG-1a and EBV-B cells as third party cells, respectively. In detail, 5x10⁴ allogeneic resting T cells and 5x10⁴ Mitomycin C-inactivated normal EBV-B cells were incubated for 6 days with various numbers of inactivated EBV-B, KG-1a and CS-1 cells as third party cells, respectively. Microcultures received [6-³H]thymidine during the last 18 hours. The ration of third party to EBV-B cells was 0.4:1, 1.3:1 or 4:1.

CS-1 cells caused dose-dependent inhibition of EBV-B-induced T cell proliferation (55.6 and 10.3 %), Figure 5. T cell proliferation was lower compared to control cultures with EBV-B and KG-1a as third party cells. Therefore, active immunosuppression rather than steric interference appears to account for reduced T cell proliferation. If CS-1 was used as single stimulator, the maximum of [6-3H]thymidine uptake was similar to that of KG-1a (4,278 ± 342 cpm versus 3,929 ± 451 cpm) which was about 8-fold lower compared to stimulation by EBV-B cells (32,632 ± 655 cpm). Thus CS-1 cells are poorly immunogeneic, which may at least partially be related to downregulation of costimulatory molecules and, in addition, CS-1 cells exert active immunosuppression.

### 7. Differentiation of CS-1 into DCs

To investigate whether CS-1 has the capacity to differentiate *in vitro,* CS-1 cells and non-malignant CD34⁺ progenitor cells, respectively, were incubated with 10 ng/ml interleukin-4 (IL-4; R&D Systems, Minneapolis, MN, USA), 100 ng/ml GM-CSF (Novartis, Basel, Switzerland) and 10 ng/ml tumor necrosis factor-α (TNF-α; WAK Chemie, Bad Homburg, Germany) according to Narita *et al.* (Exp. Hematol., 29: 709-719, 2001). These cytokines were applied alone and in a cocktail containing all of them. Cells were maintained in 24-well tissue culture plates with standard culture medium at 37 °C and 5.5 % CO₂. Cell morphology was examined by inverse phase-contrast microscopy (Wild Leitz, Heerbrugg, Switzerland) before half-medium and cytokine exchange on every fourth day. After 10 days the total amount of viable cells expressing the progenitor marker *c-kit* and the DC marker CD1a, respectively, was determined by flow cytometry as described above.

Single cytokines did not affect cell morphology. The CS-1 cell numbers in cultures with medium alone, IL-4 and GM-CSF increased by about 2.5-fold, Figure 6. Up to 99 % of the cells retained their *c-kit*^{hi}^{*+*} phenotype. TNF-α alone caused substantial cell necrosis resulting in viable cell loss from initially 2x10⁵ to 5x10⁴ + 3x10³ cells per culture after 10 days. In contrast, about 40 % of those cells cultured in the cocktail containing all cytokines developed long hair-like cytoplasmatic projections. Although the total cell amount did not change during the 10 days of culture about 50 % viable cells lost *c-kit* expression. The pronounced shape change of CS-1 and the loss of progenitor characteristics were accompanied by an upregulation of CD1a specific for DC subsets. CD80 and CD86 were also upregulated during CS-1 differentiation from 8.0 to 34.5 % and from 14.8 to 73.6 % positive cells in bulk culture, respectively. This increase was accompanied by an intensification of the mean fluorescence (CD80: 7.3 to 32.4 aU; CD86: 11.0 to 62.2 aU).

### 8. Differentiation of CS-1 into DCs during autologous T cell activation

The capacity of CS-1 to differentiate *in vitro* was examined further during autologous T cell activation. Therefore, thawed PBMNC obtained at the time of relapse and containing less than 1 % T cells and more than 95 % leukemic blasts were cultured with 100 ng/ml soluble anti CD3 mAb (clone OKT3; Cilag, Sulzbach, Germany) and 50 U/ml IL-2, or with 100 ng/ml of an irrelevant murine immunoglobulin G2a (Ig G2a/clone 20102.1; R&D Systems) as control. All cells were maintained in 24-well tissue culture plates with standard culture medium at 37 °C and 5.5 % CO₂. Cell morphology was examined by inverse phase-contrast microscopy (Wild Leitz, Heerbrugg, Switzerland) before half-medium and cytokine exchange on every fourth day. Periodically collected cells were analyzed by flow cytometry as described above.

A similar effect on CS-1 differentiation as observed above was observed during autologous T cell activation, Figure 7. Although there was a 3.5-fold increase in total cell number, *c-kit*⁺ cells failed to proliferate. Autologous T cells did not efficiently expand, as manifested by less than 20 % of T cells during the whole culture period. Beginning at day 13, differentiation into DCs resulted in an average ratio of T cells : CD1a⁺ DC of 1:3. Cultures could not be sustained beyond day 30. A 20-fold cell expansion within 27 days was recorded in control cultures with an irrelevant murine IgG2a antibody. This expansion nearly exclusively was due to *c-kit*^{hi}^{*+*} blasts. T cells did not proliferate.

### 9. Activation of allogeneic T cells by CS-1 differentiated into DCs

The question whether DC-differentiation increases the immunogenicity of CS-1 was addressed. In a first test, DC-generation cultures were derived from CS-1 cells and from normal CD34⁺ progenitor cells as described above in a medium containing the cytokine cocktail of 10 ng/ml IL-4, 100 ng/ml GM-CSF and 10 ng/ml TNF-α. Expression of various antigens on CD1a⁺ or CD83⁺ cells was measured by flow cytometry.

During the incubation period, starting CS-1 cell numbers decreased, whereas normal CD34⁺ progenitor cell cultures showed a 2.7-fold increase in the cell number. The DC fraction on day 10 was 21.8 % in CS-1-derived cultures, and 9.9 % when normal CD34⁺ progenitor cells were used. Expression levels of costimulatory and of HLA molecules were comparable on both DC types (Table 4).

**Table 4:**

| Expression of T cell-costimulatory molecules on cytokine-induced normal and malignant DCs. | | |
|---|---|---|
| | Positive DCs derived from [%] | |
| Antigen | CS-1 | Normal CD34⁺ |
| CD54 (ICAM-1) | 100.0 | 99.4 |
| CD58 (LFA 3) | 95.5 | 81.0 |
| CD80 (B7/BB1) | 74.4 | 81.6 |
| CD86 (B70/B-2) | 89.0 | 95.5 |
| HLA-DR | 85.8 | 86.5 |
| HLA-class I | 98.1 | 99.0 |

In a further test, undifferentiated CS-1 cells, CS-1-derived DCs, CD34⁺ progenitor-derived DCs and normal EBV-B cells were used as stimulator cells in MLRs using HLA non-matched allogeneic T cells as responders. In detail, 5x10⁴ allogeneic responder T cells and increasing concentrations of stimulating cells were incubated with 200 µl standard culture medium in round bottom 96-well tissue culture plates. Microcultures received 0.5 µCi [6-³H]thymidine during the last 18 of 96 hours total and [6-³H]thymidine uptake was detected as described above.

EBV-B cells and DCs derived from normal CD34⁺ progenitors caused pronounced T cell proliferation with a comparable maximum level of [6-³H]thymidine uptake (123,913 ± 2,826 cpm versus 111,983 ± 2,860 cpm), Figure 8. Over a wide range of the dose-response curve with various stimulating cell numbers, there was no difference between undifferentiated CS-1 and CS-1-derived CDs in terms of their potential to stimulate T cells. Compared to non-malignant DCs obtained from CD34⁺ progenitor cells or to normal EBV-B cells, this potential was minimal. These findings suggest that DC-differentiation alone cannot correct the poor immunogenicity of CS-1.

### 10. Tumor establishment in NOD/SCID mice

Male, non-leaky NOD/SCID mice (Jackson Laboratories, Bar Harbor, ME, USA) were bred under pathogen-free conditions. Mice received a standard diet (Sniff, Soest, Germany) and acidified water *ad libitum.*

CS-1 cells at day 909 from *in vitro*-culture or cells from suspended tumor tissue were washed and collected in cold medium without additives. Single-cell suspensions were mixed with Matrigel (Basement Membrane Matrix 40234; Becton Dickinson) as previously described (Möbest et al., Stem Cells, 17: 152-161, 1999).

To test the ability of CS-1 cells to establish *in vivo*-tumor growth, 5x10⁶ cells were injected subcutaneously (*s.c.*) into the lateral abdomen. Tumor cells from these primary tumors were passed sequentially in NOD/SCID mice. Once a week, body weight was determined. Palpable solid tumor growth was calculated using the following formula: tumor volume = (tumor width² x tumor length)/2. Mice were sacrificed after 2-4 months by cervical dislocation. Tumor, bone marrow and spleen cell suspensions, respectively, were subjected to flow cytometry using mAbs specific to human antigens as described above. Non-treated mice served as negative controls.

A typical growth curve obtained with cells from the second passage is shown in Figure 9A. After an initial latency period of more than 40 days, five of six mice developed palpable solid *s.c*.-tumors. With repeated *in vivo*-passages of either cryopreserved or freshly explanted tumor cells, latency periods shortened significantly (fresh: 35 versus 23 days; cryopreserved: 60 versus 40 days). Solid tumors explanted four months after inoculation of cells from a primary mice-borne tumor were about 0.5 to 1.2 cm in diameter. Single cell suspensions of secondary tumors mainly consisted of human cells showing the authentic CS-1 phenotype with high expression of CD34 and *c-kit* (Figure 9B). Although the spleen macroscopically was not affected, CS-1 cells disseminated into spleen and bone marrow with a frequency of 0.14 and 0.29 %, respectively, well above the detection level of flow cytometry. The phenotype of CS-1 *in vivo* was stable over time. Mice, which obtained CS-1 intravenously or intraperitoneally remained tumor free.

## Claims

1. A mammalian permanent growth factor independent-cell line constitutively overexpressing the tyrosine kinase receptor *c-kit* and/or proteins derived of *c-kit*.

2. A cell line according to claim 1, derived from a myeloid leukemia patient.

3. A cell line according to claim 2, derived from an acute myeloid leukemia (AML) patient.

4. A cell line according to claim 3, wherein the cell line is derived from a patient with an AML of the subtype M5a according to the French American British classification.

5. The cell line according to any one of the preceding claims, **characterized in that** at least 80% of the cells have the ability to express *c-kit*.

6. The cell line according to claim 5, **characterized in that** at least 85% of the cells, particularly at least 90 % of the cells have the ability to express *c-kit*.

7. The cell line according to any one of the preceding claims, wherein the *c-kit* overexpression ranges in the third decade of the mean fluorescence intensity value, as determined by flow cytometry using antibodies directly conjugated to phycoetythrin.

8. The cell line according to any one of the preceding claims, having the complex karyotype monosomy 7, t(2;11) (q31:p13), t(10;12) (q24;q24).

9. The cell line according to any one of the preceding claims, having poor immunogenicity, particularly not having the ability of substantially activate T cells.

10. The cell line according to any one of the preceding claims and proteins derived of this cell line involved in or underlying active immunosuppression.

11. The cell line according to claim 10 particularly having the ability of suppressing T cell proliferation, when the cell line is used as a third party stimulator.

12. The cell line according to any one of the preceding claims having the ability of differentiating into dendritic cells (DCs) in the presence of cytokines.

13. The cell line according to claim 12 having the ability of differentiating into DCs in the presence of recombinant cytokines.

14. The cell line according to any to claim 12 having the ability of differentiating into DCs in the presence of cytokines produced by stimulated T-cells.

15. The cell line according to claim 11 not having the ability of substantially activating T cells in its differentiated state.

16. The CS-1 cell line according to any one of the preceding claims, wherein the cell line is the cell line deposited at the Deutsche Sammlung von Mikroorganismen und Zellkulturen (DSMZ) under deposit No. ACC 2534.

17. Use of the cell line according to any one of the claims 1 to 16 for producing and purifying the tyrosine kinase receptor *c-kit* and attached signaling proteins.

18. Use of the cell line according to any one of the claims 1 to 16 for screening drugs being capable of targeting the *c-kit* signaling pathway.

19. Use of a cell line according to any one of the claims 1 to 16 for establishing tumors in mammalians and for screening agents acting with tumor cells *in vivo* and/or *in vitro.*

20. Method of producing the cell line according to any one of the claims 1 to 16 comprising
(a) obtaining a whole blood sample of a mammalian individual,
(b) separating peripheral blood mononuclear cells (PBMNC) from the whole blood sample,
(c) inoculation and continuously culturing of the PBMNC in an enriched culture medium for a first period,
(d) replacing the enriched growth medium by a standard culture medium and continuously culturing the cells for a second period of time.

21. Method according to claim 20, wherein the mammalian individual is a human patient with AML, preferably with AML of the subtype M5a.

22. Method according to claim 21, wherein the whole blood sample is obtained from a human patient after a relapse following stem cell transplantation of a matched unrelated donor or from a sibling.

23. Method according to any one of the claims 20 to 22 wherein step (d) is followed by
(e) further continuously culturing the cells at a lowered inoculum cell density in the standard culture medium for a third period of time.

24. Method according any one of the claims 20 to 23 wherein continuously culturing of the cells is conducted over a total period of time of at least 800 days, particularly at least 900 days.
